(19) Europäisches Patentamt
European Patent Office
Office européen des brevets

(11) **EP 4 289 352 A1**

(12) **EUROPEAN PATENT APPLICATION**
published in accordance with Art. 153(4) EPC

(43) Date of publication:
**13.12.2023 Bulletin 2023/50**

(51) International Patent Classification (IPC):
**A61B 5/08** (2006.01)     **A61B 5/00** (2006.01)
**A61M 16/00** (2006.01)

(21) Application number: **21923637.9**

(22) Date of filing: **08.02.2021**

(52) Cooperative Patent Classification (CPC):
**A61B 5/00; A61B 5/08; A61M 16/00**

(86) International application number:
**PCT/BR2021/050060**

(87) International publication number:
**WO 2022/165567 (11.08.2022 Gazette 2022/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Magnamed Tecnologia Médica S/A 04053-030 São Paulo (BR)**

(72) Inventors:
• **UEDA, Julio Akira**
  **São Paulo (BR)**
• **UEDA, Wataru**
  **São Paulo (BR)**
• **KINJO, Toru Miyagi**
  **São Paulo (BR)**
• **SUZUKI, Tatsuo**
  **São Paulo (BR)**

(74) Representative: **CAPRI**
  **33 rue de Naples**
  **75008 Paris (FR)**

(54) **METHOD FOR ESTIMATING THE MUSCLE PRESSURE OF A PATIENT BEING VENTILATED BY A PULMONARY VENTILATOR AND AUXILIARY DEVICE FOR A PULMONARY VENTILATOR**

(57) The present invention relates to an auxiliary device and to a method for estimating the muscle pressure of a patient being ventilated by a pulmonary ventilator, comprising: creating the database that correlates data on pressure, volume and flow of pulmonary ventilator respiratory cycles with muscle pressure data corresponding to the pressure, volume and flow data of the ventilator; using the database to create a training data set; training a predictive time-series algorithm with the training data set; populating the algorithm with pressure, volume and flow data from a pulmonary ventilator, in which the pressure, volume and flow data include at least a time series with pressure, volume and flow curve points; and using the algorithm to estimate the muscle pressure value for at least one of the time series points.

**FIG. 2**

**Description**

FIELD OF THE INVENTION

The present invention refers to a method for estimating the muscle pressure of a patient being ventilated by a pulmonary ventilator and a device trained by machine learning using the method of the present invention.

BACKGROUND OF THE INVENTION

A mechanically ventilated patient falls generically into two different situations: the patient being sedated, such that all the inspiration and expiration is controlled by the mechanical ventilator; and the patient interacting with the mechanical ventilator, making his or her own effort and activating the start of an inspiration assisted by the mechanical ventilator.

In controlled ventilation, the triggering of the ventilator is commanded by time, with programmed adjustment of the respiratory frequency. In assisted ventilation, wherein the patient exerts some effort, the respiratory cycle is started by said effort. Normally, the respiratory cycle is divided into four phases: the inspiratory phase, in which the lung inflates, a change from the inspiratory phase to the expiratory phase, the expiratory phase, in which the lungs are emptied, and a change from the expiratory to the inspiratory phase.

In the change from expiratory to inspiratory phase, the ventilator is triggered and in the change from inspiratory to expiratory phase ventilator cycling occurs. During the inspiratory phase, the lung is inflated, and during the expiratory phase, it is passively emptied.

In situations wherein the patient exerts some effort, there is a greater risk of patient / ventilator asynchronicities because the ventilation modalities used in these situations are sensitive to the patient's muscular stimuli. By and large, asynchronicities can be defined as moments or situations of incoherence between a patient's requirements and the offer of a mechanical ventilator, be this relating to time, flow, volume and/or pressure of the respiratory system.

The most common asynchronicities are those referred to as "self-triggering", when the ventilator wrongly understands that the patient wishes to breathe in, and triggers an inspiratory cycle, and the "lost effort", when the ventilator fails to recognize the patient's effort and the patient receives no support from the ventilator.

A patient's inspiratory requirement is normally associated to his or her effort, which, in turn, is normally measured by muscle pressure (Pmus).

In a mechanically ventilated patient, the respiratory pressure generally abides by the following equation:

$$P_{mus} + P_{ventilator} = \text{flow x resistance} + \text{volume x elastance}$$

The operating pressure, flow and volume of the ventilator are known magnitudes. Resistance, elastance and muscle pressure are magnitudes that depend on the patient's respiratory system, and elastance is oftentimes defined as the opposite of compliance.

Although some methods for measuring muscle pressure with the use of catheters are known, these methods from the state of the art require invasive and complex procedures.

Knowing the Pmus value, either by measurement or estimation, has various advantages. On the one hand, it enables, for example, improved adjustment of the operating parameters of the ventilator and better ventilator-patient synchronicity, while on the other hand it enables the resistance, elastance and compliance measurements to be calculated.

The resistance (R) and compliance (C) measurements in sedated patients can be obtained directly by monitoring the mechanical ventilation. The R and C values cannot be calculated in a simple way in the presence of Pmus.

By accompanying the resistance and compliance values, it is possible to monitor the performance of a patient's respiratory system, track the progression of diseases and identify complications such as bronchospasms and pulmonary edema.

So the need persists in the state of the art for a method that allows the estimation of the muscle pressure of a patient assisted by a pulmonary ventilator that is efficient, but less complex and invasive than the measurement and estimation methods known in the state of the art.

OBJECTIVES OF THE INVENTION

It is one of the objectives of the present invention to provide a method for estimating the muscle pressure of a patient being ventilated by a pulmonary ventilator that is effective, but not invasive.

It is another of the objectives of the present invention to provide a method for estimating the muscle pressure of a patient being ventilated by a pulmonary ventilator using operating magnitudes of the mechanical ventilator.

It is yet another of the objectives of the present invention to provide a method for estimating the muscle pressure of a patient being ventilated by a pulmonary ventilator using the flow, pressure and volume magnitudes of the mechanical ventilator.

It is a further one of the objectives of the present invention to provide a method for estimating the muscle pressure of a patient being ventilated by a pulmonary ventilator using machine learning.

Another of the objectives of the present invention is to provide an auxiliary device trained by the method of the present invention, that allows a patient's Pmus curve to be obtained from any pulmonary ventilator.

BRIEF DESCRIPTION OF THE INVENTION

The present invention achieves the above objectives by way of a method for estimating the muscle pressure of a patient being ventilated by a pulmonary ventilator, comprising:

creating a database that correlates data on pressure, volume and flow of pulmonary ventilator respiratory cycles with muscle pressure data corresponding to said pressure, volume and flow data of the ventilator;
using the databased to create a training data set;
training a predictive time-series algorithm with the training data set;
populating the algorithm with pressure, volume and flow data from a pulmonary ventilator, the pressure, volume and flow data comprising at least one time series with pressure, volume and flow curve points generated by the ventilator; and
using the algorithm to estimate the muscle pressure value for at least one of the time series points.

In one embodiment of the present invention, the predictive time-series algorithm is a recurrent neural network LSTM (Long Short-Term Memory), and the database covers data obtained with a pulmonary ventilator connected to a breathing simulator, the breathing simulator enabling the simulation of different resistance and elastance parameters.

In another embodiment, the database covers data obtained with a breathing simulator and data obtained with a pulmonary ventilator in operation.

The populating step the algorithm with pressure, volume and flow data from a pulmonary ventilator may comprise populating data relating to a point, taken in a same time period, in each of the pressure, volume and flow curves and also forty points on each side of said points taken in the same time period.

Preferably, the method of the present invention is used to estimate points of a muscle pressure curve.

The method of the present invention may further comprise a post-processing step wherein a two-order Savitzky-Golay filter is applied to the muscle pressure curve.

The present invention further encompasses a system for estimating the muscle pressure of a patient being ventilated by a pulmonary ventilator, comprising:

at least one computer running a predictive time-series algorithm trained with a training data set obtained from a database that correlates data on pressure, volume and flow of pulmonary ventilator respiratory cycles with muscle pressure data corresponding to the pressure, volume and flow data of the ventilators; and
a pulmonary ventilator generating pressure, volume and flow data of a pulmonary ventilator, the pressure, volume and flow data comprising at least one time series with pressure, volume and flow curve points generated by the ventilator;
wherein the pressure, volume and flow data generated by the pulmonary ventilator are populated to the algorithm; and
wherein the algorithm estimates a muscle pressure value for at least one of the time series points.

In one embodiment of the system of the present invention, the predictive time-series algorithm is a recurrent neural network LSTM (Long Short-Term Memory), and the database covers data obtained with a pulmonary ventilator connected to a breathing simulator, the breathing simulator enabling the simulation of different resistance and elastance parameters, with tools for generating the Pmus curve having adjustable amplitude and duration.

In one embodiment of the system, the database covers data obtained with a breathing simulator and data obtained with a pulmonary ventilator in operation.

In the system of the present invention, the algorithm is also populated with the data relating to a point, taken in a same time period, in each of the pressure, volume and flow curves and also forty points on each side of said points taken in the same time period.

BRIEF DESCRIPTION OF THE DRAWINGS

The present invention will now be described in further detail, with references to the accompanying drawings, wherein:

Figure 1 - is an illustrative chart depicting the pressure curves, flow and volume of a mechanical ventilator;
Figure 2 - is an illustrative chart that compares the pressure curves, flow and volume of a mechanical ventilator with the muscle pressure curves of a patient;
Figure 3 - is a schematic representation of the method for estimating the muscle pressure of according to the present invention;
Figure 4 - is a schematic representation of a preferred embodiment of the method for estimating the muscle pressure of according to the present invention;
Figure 5 to 9 - are representations of neural networks for application with the method according to the present invention.

DETAILED DESCRIPTION OF THE INVENTION

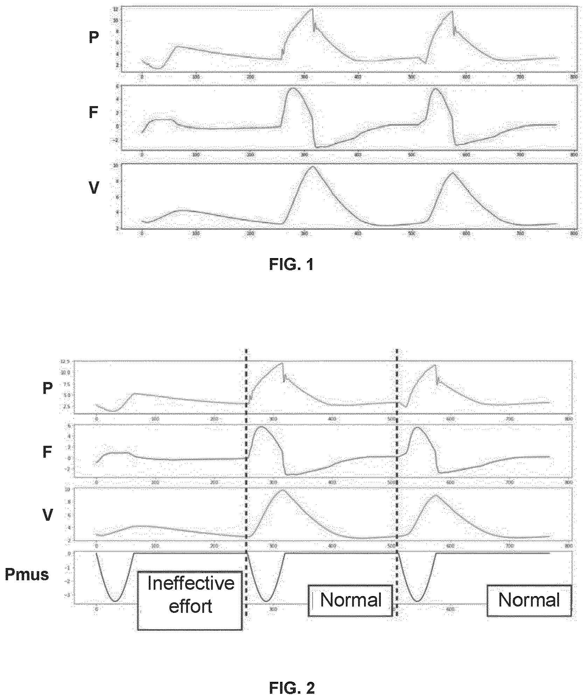

The present invention will now be described based on an embodiment of the invention illustrated in figures 1 to 9.

Figure 1 shows the curves of the pressure, flow and volume measurements of a patient connected to a mechanical ventilator.

As better illustrated in figure 2, which includes the Pmus (muscle pressure) measurement, the patient's effort is reflected in the Pmus curve as a negative curve. In assisted ventilation, the patient's effort should activate the start of an inspiration by the mechanical ventilator. When this does not happen, there is an ineffective effort, an asynchronicity between patient and ventilator.

Therefore, in the curves shown in figures 2, the first negative Pmus curvature represents an ineffective effort of the patient, because, as made clear from the pressure curves, flow and volume of the ventilator, there was no activation of same for the start of the cycle. In the following two efforts, the ventilator was activated and no asynchronicities were noted.

The correct evaluation of the patient's effort generates benefits not only for the adjustment and control of the ventilator, but also for correct evaluation of the patient, both in terms of diagnosis and in therapeutic terms.

The present invention proposes a method for estimating the muscle pressure of a patient connected to a pulmonary ventilator, which uses machine learning to predict a muscle pressure curve (Pmus).

As known by persons skilled in the art, the machine learning involves an algorithm that is trained, validated and tested.

One of the technical problems involved in the present invention is precisely the capacity to predict / estimate the muscle pressure of a patient assisted by a pulmonary ventilator based on data readily available in the pulmonary ventilator.

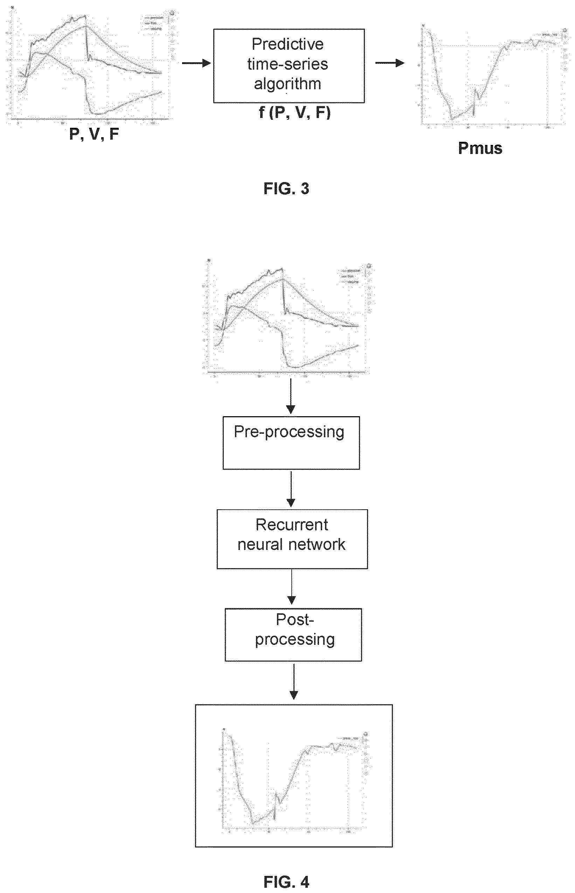

As schematically illustrated in figure 3, to meet the technical requirement, the method of the present invention has to be capable of estimating the muscle pressure (Pmus) based on pressure, volume and flow data (P, V, F) commonly available in a pulmonary ventilator, be it mechanical or electronic.

Accordingly, in the method of the present invention, a database is built that that correlates data on pressure, volume and flow from respiratory cycles of pulmonary ventilator with muscle pressure data corresponding to said pressure, volume and flow data of the ventilator.

The database is preferably built through simulations carried out with a breathing simulator connected to a pulmonary ventilator. In modelling the present method, the breathing simulator used was the ASL 5000™ simulator by the company IngMar Medical.

Accordingly, the simulator was programmed to generate a plurality of respiratory cycles with variability of effort (Pmus values) and mechanical respiration parameters (resistance and compliance) and the ventilator was programmed to generate diverse forms of waves (or time series) of pressure, flow volume based on the various combinations of resistance and compliance values.

Since each different type of asynchronicity - self-triggering, double-triggering, ineffective effort, etc. - significantly influences the muscle pressure, the database needs to include the different types of possible asynchronicities for assisted ventilation generated by different parameters of resistance and compliance. In modelling the method of the present invention, the database was created from 2067 cycles simulated for the training set and 918 cycles simulated for validating the time-series prediction algorithm. The database also included 363 cycles of a ventilator in operation for testing the algorithm.

The training and test sets were sorted so as to assure that each set included cycles with different resistance and compliance values. Effort was also made to assure that the sets included cycles with corresponding Pmus values with different widths and amplitudes, so as to increase the variability of the data.

As better illustrated in Figure 4, in the preferred embodiment of the present invention, the time-series prediction algorithm is a recurrent neural network bidirectional LSTM (Long Short Term Memory), and a pre-processing step is performed on the input data, wherein, for each point taken in a same time period on each pressure curve, volume and flow which will be populated to the neural network, the forty points on each side of said point are also populated.

The population of the points on each side of the time series is intended to give the neural network the context necessary for prediction.

For estimating the Pmus curve, the algorithm estimates the muscle pressure value for various time series points, so that the curve can be predicted.

In one embodiment of the invention, the estimated muscle pressure curve also undergoes a post-processing with a two-order Savitzky-Golay filter, so as to decrease the intensity of potential errors in this forecast.

Therefore, the objective of the filter applied is to temper a digital signal, that is, decrease the noise without causing distortion, and acts through convolution processes, approximating the points near to polynomials by way of the least squares method. After passing through the filter, the Pmus curve is capable of assisting in the correct evaluation of the effort by a patient connected to a pulmonary ventilator.

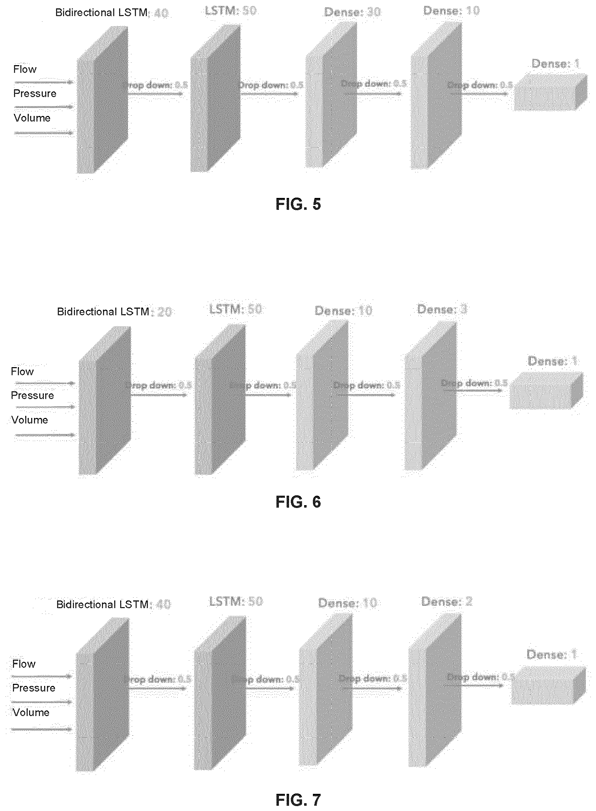

Figures 5 to o show examples of neural networks developed for application in the method of the present invention.

Therefore, figure 5 shows the structure of the neural network for a pressure-controlled ventilation (PCV) mode. As shown in the figure, the neural network is comprised of a first layer of Bidirectional LSTM with 40 memory units, a second layer of traditional LSTM with 50 memory units and three Dense layers with the respective neuron numbers: 30, 10 and 1. Additionally, a dropout regularization is implemented between each layer of the neural network to prevent overadjusting the model to the data.

Figure 6 shows the structure of the neural network for a volume controlled ventilation (VCV) mode. As shown in the figure, the neural network is comprised of a first layer of Bidirectional LSTM with 20 memory units, a second layer of traditional LSTM with 50 memory units and thereafter three Dense layers with the respective neuron numbers: 10, 3 and 1. Additionally, a dropout regularization function is implemented between each layer of the neural network to prevent overadjusting the model to the data.

Figure 7 shows the structure of the neural network for a pressure-synchronized intermittent mandatory ventilation (PSIMV) mode. As shown in the figure, the neural network is comprised of a first layer of Bidirectional LSTM with 40 memory units, a second layer of traditional LSTM with 50 memory units and three Dense layers with the respective neuron numbers: 10, 2 and 1. Additionally, a dropout regularization is implemented between each layer of the neural network to prevent overadjusting the model to the data.

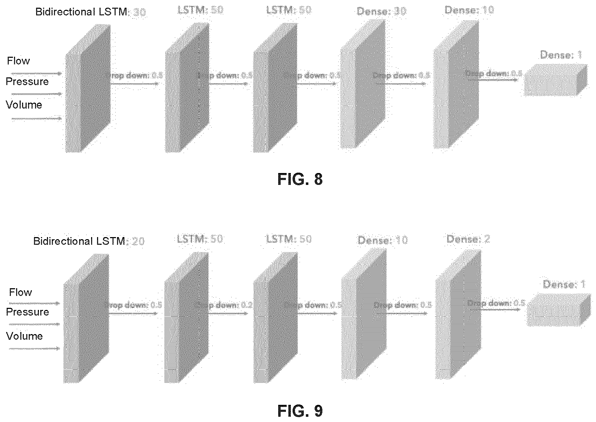

Figure 8 shows the structure of the neural network for a volume-synchronized intermittent mandatory ventilation (VSIMV) mode. As shown in the figure, the neural network is comprised of a first layer of Bidirectional LSTM with 30 memory units, two layers traditional LSTM sequenced with 50 memory units and thereafter three Dense layers with the respective neuron numbers: 30, 10 and 1. Additionally, a dropout regularization function is implemented between each layer of the neural network to prevent overadjusting the model to the data.

Figure 9 shows the structure of the neural network for a pressure support ventilation (PSV) mode. As shown in the figure, the neural network is comprised of a first layer of Bidirectional LSTM with 40 memory units, two layers traditional LSTM sequenced with 50 memory units and thereafter three Dense layers with the respective neuron numbers: 10, 2 and 1. Additionally, a dropout regularization is implemented between each layer of the neural network to prevent overadjusting the model to the data.

The present invention also encompasses a device trained with the method of the present invention.

The device according to the present invention can be used outside (connected) or integrated to the pulmonary ventilator, so as to monitor the pressure, volume and frequency of the ventilator to estimate the Pmus of the ventilated patient, calculating the resistance and compliance values and accompanying the performance of the patient's respiratory system.

Therefore, in a preferred embodiment, the device comprises a flow and pressure sensor that measures the gas entering and leaving the patient's mouth and the pressure, volume and flow data are used as input in the Pmus estimation method.

In one embodiment of the present invention, the device is connected to a vital signs monitor.

Having described an example of a preferred embodiment of the present invention, it should be understood that the scope of the present invention encompasses other potential variations of the inventive concept described, being limited solely by the content of the claims, with possible equivalents included therein.

**Claims**

1. A method for estimating the muscle pressure of a patient being ventilated by a pulmonary ventilator, **characterized**

**by** comprising:

creating the database that correlates data on pressure, volume and flow of pulmonary ventilator respiratory cycles with muscle pressure data corresponding to said pressure, volume and flow data of the ventilator; using the database to create a training data set; training a predictive time-series algorithm with the training data set; populating the algorithm with pressure, volume and flow data from a pulmonary ventilator, the pressure, volume and flow data comprising at least one time series with pressure, volume and flow curve points generated by the ventilator; and using the algorithm to estimate the muscle pressure value for at least one of the time series points.

2. The method according to claim 1, **characterized in that** the predictive time-series algorithm is an LSTM (Long Short-Term Memory) recurrent neural network.

3. The method according to claim 1 or 2, **characterized in that** the database covers data obtained with a pulmonary ventilator connected to a breathing simulator, the breathing simulator enabling the simulation of different resistance and elastance parameters and the introduction of muscle pressures having adjustable amplitude and duration.

4. The method according to claim 3, **characterized in that** the database covers data obtained with a breathing simulator and data obtained with a pulmonary ventilator in operation.

5. The method according to any of claims 1 to 4, **characterized in that** the populating step the algorithm with pressure, volume and flow data from a pulmonary ventilator comprises populating the data relating to a point, taken in a same time period, in each of the pressure, volume and flow curves and also forty points on each side of said points taken in the same time period.

6. The method according to claim 5, **characterized in that** the estimating step comprises estimating points of a muscle pressure curve.

7. The method according to claim 6, **characterized by** comprising a post-processing step wherein a two-order Savitzky-Golay filter is applied to the muscle pressure curve.

8. An auxiliary device for a pulmonary ventilator, **characterized by** being trained with the method defined in any of claims 1 to 7.

9. The device according to claim 8, **characterized by** being connectable to the pulmonary ventilator.

10. The device according to claim 8, **characterized by** being integrated to the pulmonary ventilator.

11. The device according to claim 8 or 9, **characterized by** being integrated to a vital signs monitor.

FIG. 1

FIG. 2

Predictive
time-series
algorithm
**f (P, V, F)**

**P, V, F**

**Pmus**

**FIG. 3**

Pre-processing

Recurrent
neural network

Post-
processing

**FIG. 4**

**FIG. 5**

**FIG. 6**

**FIG. 7**

FIG. 8

FIG. 9

## INTERNATIONAL SEARCH REPORT

| International application No. |
|---|
| PCT/BR2021/050060 |

### A.  CLASSIFICATION OF SUBJECT MATTER

**IPC: A61B5/08 (2006.01), A61B5/00 (2006.01), A61M16/00 (2006.01)**
**CPC: A61B5/08, A61B5/7221, A61M16/026**

According to International Patent Classification (IPC) or to both national classification and IPC

### B.  FIELDS SEARCHED

Minimum documentation searched (classification system followed by classification symbols)

**E61B, A61M**

Documentation searched other than minimum documentation to the extent that such documents are included in the fields searched

**Base de Patentes INPI-BR**

Electronic data base consulted during the international search (name of data base and, where practicable, search terms used)

**DERWENT. GOOGLE PATENTS**

### C.  DOCUMENTS CONSIDERED TO BE RELEVANT

| Category* | Citation of document, with indication, where appropriate, of the relevant passages | Relevant to claim No. |
|---|---|---|
| X | EP 3391816 B1 ( CONVERGENT ENGINEERING INC [US]) 09 September 2020 (09.09.2020) (abstract; Fig. 9; par. [0020] – [0033], [0060], [0061], [0067], [0069] – [0072]) | 1 – 11 |
| X | WO 2013126417 A1 (UNIV FLORIDA [US]) 29 August 2013 (29.08.2013) (abstract; Figs. 2, 4, 5; p. 4, l. 20 – 27; p. 5, l. 9 – p. 6, l. 32; p. 11, l. 11 – p. 12, l. 14; p. 14, l. 1 – 34) | 1 – 11 |
| Y | US 10828437 B2 (COVIDIEN LP [US]) 10 November 2020 (10.11.2020) (abstract; Figs. 4, 6; col. 2, l. 33 – col. 3, l. 9; col. 5, l. 9 – col. 6, l. 5; col. 6, l. 43 – col 7, l. 12; col. 14, l. 7 – 31) | 1 – 11 |

[X] Further documents are listed in the continuation of Box C.       [X]   See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 28 May 2021 (28.05.2021 | 07 June 2021 (07.06.2021) |

| Name and mailing address of the ISA/ BR | Authorized officer |
|---|---|
| | |
| Facsimile No. | Telephone No. |

Form PCT/ISA/210 (second sheet) (January 2015)

**INTERNATIONAL SEARCH REPORT**

International application No.

**PCT/BR2021/050060**

| C (Continuation). | DOCUMENTS CONSIDERED TO BE RELEVANT | |
|---|---|---|
| Category* | Citation of document, with indication, where appropriate, of the relevant passages | Relevant to claim No. |
| Y | US 8457706 B2 ( COVIDIEN LP [US])<br>4 June 2013 (04.06.2013)<br>(abstract; Figs. 2 – 5; col. 1, l. 49 – col 2, l. 51; col. 8, l. 9 – col. 11, l. 16)<br>------------------------------------------- | 1 – 11 |

Form PCT/ISA/210 (continuation of second sheet) (January 2015)

**INTERNATIONAL SEARCH REPORT**
Information on patent family members

| International application No. |
|---|
| **PCT/BR2021/050060** |

| | | | |
|---|---|---|---|
| EP 3391816 B1 | 2020-09-09 | EP 3391816 A1 | 2018-10-24 |
| | | BR 112012016102 B1 | 2020-11-03 |
| | | CN 102770070 A | 2012-11-07 |
| | | EP 2519151 A2 | 2012-11-07 |
| | | JP 2013515592 A | 2013-05-09 |
| | | JP 5858927 B2 | 2016-02-10 |
| | | JP 2016104154 A | 2016-06-09 |
| | | JP 6564318 B2 | 2019-08-21 |
| | | US 2012330177 A1 | 2012-12-27 |
| | | US 8728002 B2 | 2014-05-20 |
| | | WO 2011090716 A2 | 2011-07-28 |
| WO 2013126417 A1 | 2013-08-29 | AU 2003268324 A1 | 2004-03-19 |
| | | BR 0313823 A | 2005-07-05 |
| | | BR PI0313823 B1 | 2020-03-10 |
| | | CA 2492528 A1 | 2004-03-11 |
| | | CA 2861505 A1 | 2013-08-29 |
| | | CN 104135925 A | 2014-11-05 |
| | | EP 1534131 A2 | 2005-06-01 |
| | | EP 2377463 A1 | 2011-10-19 |
| | | EP 2816952 A1 | 2014-12-31 |
| | | JP 2005537068 A | 2005-12-08 |
| | | JP 4836453 B2 | 2011-12-14 |
| | | US 2004040560 A1 | 2004-03-04 |
| | | US 7425201 B2 | 2008-09-16 |
| | | US 2007232951 A1 | 2007-10-04 |
| | | US 7588543 B2 | 2009-09-15 |
| | | US 2009272382 A1 | 2009-11-05 |
| | | US 8617083 B2 | 2013-12-31 |
| | | US 2012215081 A1 | 2012-08-23 |
| | | US 8672858 B2 | 2014-03-18 |
| | | WO 2004019766 A2 | 2004-03-11 |
| US 10828437 B2 | 2020-11-10 | US 2018207378 A1 | 2018-07-26 |
| | | CN 102056538 A | 2011-05-11 |
| | | CN 102056539 A | 2011-05-11 |
| | | EP 2320790 A1 | 2011-05-18 |
| | | EP 2320791 A1 | 2011-05-18 |
| | | JP 2011522620 A | 2011-08-04 |
| | | JP 2011522621 A | 2011-08-04 |
| | | JP 2015096212 A | 2015-05-21 |
| | | US 2009301486 A1 | 2009-12-10 |
| | | US 8485183 B2 | 2013-07-16 |
| | | US 2009301487 A1 | 2009-12-10 |
| | | US 8485184 B2 | 2013-07-16 |
| | | US 2009301491 A1 | 2009-12-10 |
| | | US 8485185 B2 | 2013-07-16 |
| | | US 2009301490 A1 | 2009-12-10 |
| | | US 8826907 B2 | 2014-09-09 |
| | | US 2013276788 A1 | 2013-10-24 |
| | | US 9114220 B2 | 2015-08-25 |
| | | US 2013284173 A1 | 2013-10-31 |
| | | US 9126001 B2 | 2015-09-08 |

Form PCT/ISA/210 (patent family annex) (January 2015)

INTERNATIONAL SEARCH REPORT
Information on patent family members

International application No.

**PCT/BR2021/050060**

| | | | |
|---|---|---|---|
| | | US 2014345616 A1 | 2014-11-27 |
| | | US 9925345 B2 | 2018-03-27 |
| | | US 2015314089 A1 | 2015-11-05 |
| | | US 9956363 B2 | 2018-05-01 |
| | | US 2018207379 A1 | 2018-07-26 |
| | | WO 2009149351 A1 | 2009-12-10 |
| | | WO 2009149353 A1 | 2009-12-10 |
| | | WO 2009149355 A1 | 2009-12-10 |
| | | WO 2009149357 A1 | 2009-12-10 |
| US 8457706 B2 | 2013-06-04 | US 2009287070 A1 | 2009-11-19 |

Form PCT/ISA/210 (patent family annex) (January 2015)